# EUROPEAN PATENT SPECIFICATION

(11) **EP 1 658 073 B1**
(45) Date of publication and mention of the grant of the patent: **22.02.2012**
(21) Application number: 04766541.9
(22) Date of filing: 19.08.2004
(51) Int. Cl.: A61K 31/44, A61J 3/00

(54) **PHARMACEUTICAL PRODUCT FOR INJECTION**
PHARMAZEUTISCHES INJEKTIONSPRÄPARAT
PRODUIT PHARMACEUTIQUE D'INJECTION

(30) Priority: 21.08.2003 US 496715 P
(43) Date of publication of application: 24.05.2006
(73) Proprietor: Nycomed GmbH, 78467 Konstanz (DE)
(72) Inventor: LIPPERT, Rita, 78467 Konstanz (DE); LINDER, Rudolf, 78464 Konstanz (DE)
(74) Representative: Kratzer, Bernd
(86) International application number: PCT/EP2004/051841
(87) International publication number: WO 2005/018639

(56) References cited:
- US-A1- 2003 003 058
- "Section 3 - Summary of Product Characteristics" PROTIUM -PRODUCT SUMMARY. ISSUED BY BYK GOLDEN , DE, January 2000 (2000-01), XP002163617
- LEITNER ET AL: "Visuelle dokumentation der Stabilität der intravenösen Lösungen von Omeprazol und Pantaprazol" WIEN.MED.WSCHR, vol. 152, 2002, pages 568-573, XP001203762

## Description

### Technical Field

The present invention relates to the field of pharmaceutical technology and describes an improved pharmaceutical product for injection. More particular the present invention relates to improved pharmaceutical products comprising inter alia s4ifluoromethoxy-2-[(3,4-dimethoxy-2-pyridinyl)methylsulfinyl]-1H-benzimidazole preparations for injection.

### Prior art

WO94/02141 describes an injection comprising a 2-[(2-pyridyl)methylsulfinyl]-benzimidazole compound an aqueous solvent added with no nonaqueous solvent, wherein the pH of the injection is not less than 9.5 and not more than 11.5. It is mentioned that said injection does not cause hemolysis and causes less local irritation.

DE 43 24 014 describes the preparation of a lyophilisate of pantoprazole-sodium sesquihydrate in the presence of sucrose as an auxiliary at production temperatures of -25 to -30°C. It is disclosed that the lyophilisate is of improved storage stability and can be stored at room temperature for at least 18 months and is easily reconstituted in liquid form in suitable doses for use.

CN 1235018 describes a freeze-dried injection powder of pantoprazole sodium containing no crystallised water with pH value of 9-12.5, which is composed of pantoprazole sodium, freeze-dried powder supporting agent, metal ion complexing agent and pH regulator.

WO99/18959 describes aqueous pharmaceutical compositions, which are chemically and physically stable for intravenous injection which comprise anti-ulcerative compound and glycine as stabilizer in carrier.

WO02/41919 describes lyophilized pantoprazole preparations, which are obtainable by freeze-drying of an aqueous solution of pantoprazole, ethylendiamine tetraacetic acid and/or a suitable salt thereof, and sodium hydroxide and/or sodium carbonate. The preparations have advantageous properties when reconstituted for injection.

Pantoprazole sodium for injection is commercially available as a freeze-dried powder in a clear glass vial fitted with a rubber stopper and crimping seal (e.g. in the United States under the trademark Protonix® I.V.; see e.g. Physicians' Desk Reference entry for Protonix® I.V). For administration injection admixtures should be administered intravenously through a dedicated line, using an in-line filter provided. The filter must be used to remove precipitate that may form when the reconstituted drug product is mixed with I.V. solutions.

Reconstitution of lyophilised pharmaceutical compounds with carrier solutions for application may lead to the formation of visible and/or subvisible particles in the solution. Injectable solutions, including solutions constituted from sterile solids intended for parenteral use should be essentially free from particles that can be observed on visual inspection and for patient safety it is also desirable to have a low number of subvisible particles. USP (United States Pharmacopoeia) 24 describes physical tests performed for the purpose of enumerating subvisible extraneous particles within specific size ranges and also defines particulate matters limits set forth for the test being applied for large-volume injections for single-dose infusion and small-volume injections (USP 24, <788> Particulate Matter in Injections).

### Description of invention

Surprisingly it has now been found that by using container/closure systems for a pharmaceutical product comprising pantoprazole for injection, which essentially do not release zinc ions formation of particles in pantoprazole injection solutions can be suppressed or completely avoided. Without being limited to this explanation it is believed that zinc ions released from the container/closure system when coming into contact with the reconstituted pantoprazole injection solution can lead to the formation of zinc-pantoprazole particles in the injection solution. Thus by using material for the container/closure system which essentially does not release zinc ions the formation of particles in pantoprazole injection solutions can be suppressed or completely avoided.

Subject of the present invention therefore is a pharmaceutical product for injection comprising a container including a closure suitable for preparations for injection, the container containing a compound selected from the group of 5-difluoromethoxy-2-[(3,4-dimethoxy-2-pyridinyl)methylsulfinyl]-1H-benzimidazole (pantoprazole), a salt thereof, a solvate of pantoprazole and a salt thereof, wherein the container and closure are made of material which essentially does not release zinc ions.

Containers suitable for injection in connection with the invention refers to containers which do not interact physically or chemically with the preparation for injection (pantoprazole) in any manner to alter the strength, quality, or purity beyond the official requirements under the ordinary or customary conditions of handling, shipment, storage, sale and use. The container in connection with the invention is made of material, which essentially does not release zinc ions. Suitable containers in accordance with the invention are for example made of glass. Particular suitable are type I glass containers (according to European Pharmacopoeia 2002). In one embodiment of the invention the container is a clear glass vial which fulfills the criteria of type I glass containers according to European Pharmacopoeia 2002 (e.g. glass available under the name Fiolax ® - klar).

The container for injection according to the invention is closed or sealed with a suitable closure in such a manner as to prevent contamination or loss of content. In a preferred embodiment of the invention the closure permits penetration by a needle and, upon withdrawal of the needle closes at once, protecting the container against contamination. The closure in connection with the invention is made of material, which essentially does not release zinc ions. Closure material which essentially does not release zinc ions refers to material wherein the amount of extractable zinc is 0 ppm (i.e. not detectable) of extractable zinc. The amount of extractable zinc is determined according to methods specified in the European Pharmacopoeia 2002 for the determination of extractable zinc in rubber stoppers. Suitable closures are made for example from rubber or silicon elastomer. In this connection it is preferred to make use of rubber or silicion elastomer for the stopper, which was manufactured without the use of components containing zinc (e.g. without using zinc catalysts in the polymerization process). In another embodiment according to the invention it is preferred to make use of a closure, which is completely or partially laminated or coated with a suitable material. Preferably those surface parts of the closure, which may come into contact with the drug (e.g. those parts of the closure extending inside the container) are laminated or coated with a suitable inert material. As an example a suitable fluoro-polymer resin can be used for lamination. Such lamination establishes an effective closure-drug barrier and reduces or completely prevents drug-closure interaction. Further such lamination eliminates escape of endogeneous particles from the closure and drugs will be prevented to adhere to the closure. Further lamination or coating provides lubricity for machinability, thereby eliminating closure sticking or clumping problems in production. Preferably the material for the closure is type 1 rubber according to European Pharmacopoeia 2002. Preferably the closure is a freeze drying closure. Freeze drying closure in connection with the invention refers to a closure, which enables drying of a frozen good in a vacuum chamber. Such freeze drying closure is put in place on top of a glass container after filling leaving sufficient openings for the sublimation process under vacuum. At the end of the drying process it can be fully inserted into the glass container by hydraulic or mechanical means in the vacuum chamber. The plug part of such freeze drying closure preferably provides slits, channels or other appropriate means in conjunction with protruding or locating elements at the outer diameter, which enable insertion in a drying position (halfway; also referred to as semi stoppered herein) during the sublimation process. Preferably the design of the locating element to hold the freeze drying closure firmly in the sublimation positon shall not generate too high a resistance when the closure is fully inserted. On the top surface of the closure marks or indentations may be present. In a particularly preferred embodiment the closure is a butyl rubber stopper of type 1 according to European Pharmacopoeia 2002, which is partially fluoro-polymer laminated whereby lamination is covering all those parts extending inside the vial including the plug part.

In another embodiment the pharmaceutical product additionally comprises a suitable crimp seal.

In a preferred embodiment the pharmaceutical product comprises a clear glass vial fitted with a rubber stopper and crimp seal.

In another preferred embodiment the container comprises a vial with a blow back inside of the flange. The blow back improves the fit of the stopper and avoids that the stopper pops out of the vial. The flange of the vial and the dimensions of the stopper are chosen in a way to guarantee a good fit of the stopper during freeze drying process when the stopper is not completely pressed into the vial, as well as after the freeze drying process when the stopper is completely pressed into the vial and not already fixed by a crimping seal. It is preferred to have a blow back with dimensions in size to provide sufficient sealing surface between the vial and the stopper in order to keep a vacuum in the vial as long as possible. In case of a vial with a blow back a stopper is preferred which is partially laminated with a fluoro-polymer. Preferably the surface of the stopper contacting the blow back of the vial is not laminated but lamination extends from this area of the stopper downwards the plug part and covers at least all those parts extending inside the vial.

The container according to the invention preferably permits the addition of a suitable solvent and withdrawal of all or portions of the resulting injection in such a manner, that the sterility of the product is maintained. The container according to the invention may hold any suitable volume, preferably 20 ml or less, more preferably 15 ml or less, particularly preferably 12 ml or less (for example 12 ml).

In another embodiment according to the invention the closed container closure system has reduced pressure inside. Preferably the pressure is reduced to allow the addition of solvent for injection to the closed system (e.g. by means of penetrating the closure with a needle). Preferably the reduced pressure is 800 mbar or below, 600 mbar or below, in particular 500 mbar or below (e.g. 500 mbar). As compared to container closure/systems having no reduced pressure inside such container/closure systems allow the addition of sufficient solvent for injection. Container/closure systems having a blow back are particularly preferred in connection when applying reduced pressure. As compared to conventional systems having no blow back, said systems have proven to be very tight and the risk of influx of air and thus potential contamination can be avoided.

5-Difluoromethoxy-2-[(3,4dimethoxy-2-pyridinyl)methylsulfinyl]-1H-benzimidazole (INN: pantoprazole, in connection with the invention also referred to as pantoprazole) is known from EP-A-0 166 287. Pantoprazole is a chiral compound. In connection with the invention the term pantoprazole also includes the pure enantiomers of pantoprazole and their mixtures in any mixing ratio. (S)-pantoprazole [(-)-pantoprazole] may be mentioned by way of example. Pantoprazole is present here as such or preferably in the form of it's salt with a base. Examples of salts with a base which may be mentioned are sodium, potassium, magnesium and calcium salts. Pantoprazole and/or a salt thereof may contain various amounts of solvent when isolated in crystalline form. In connection with the invention pantoprazole also refers to all solvates and in particular to hydrates of 5-difluoromethoxy-2-[(3,4-dimethoxy-2-pyridinyl)methylsulfinyl]-1H-benzimidazole and salts thereof. Such a hydrate of the salt of pantoprazole with a base is disclosed, for example, in WO91/19710 Expediently pantoprazole refers to pantoprazole sodium, pantoprazole sodium sesquihydrate (= pantoprazole sodium x 1.5 H₂O), pantoprazole magnesium dihydrate and (-)-pantoprazole magnesium dihydrate (in this connection reference is made to WO00/10995 and WO04/013126).

Preferably the pantoprazole is contained as powder in the pharmaceutical product according to the invention. It is particularly preferred to provide the pantoprazole as freeze dried (herein also referred to as lyophilized) preparation.

Instead of pantoprazole the pharmaceutical product according to the invention may also contain other acid-labile proton pump inhibitors (H⁺/K⁺ ATPase inhibitors). Other acid-labile proton pump inhibitors within the meaning of the present invention which may be mentioned are in particular substituted pyridin-2-yl-methylsulfinyl-1H-benzimidazoles, such as are disclosed, for example, in EP-A-0 005 129, EP-A-0 166 287. EP-A 0 174 726, EP-A-0 184 322, EP-A-0 261 478 and EP-A-0 268 956. Mention may preferably be made here of 5-methoxy-2-[(4-methoxy-3,5-dimethyl-2-pyridinyl)methylsulfinyl]-1H-benzimidazole (INN: omeprazole), 2-[3-methyl-4-(2,2,2-trifluoroethoxy)-2-pyridinyl)methylsulfinyl]-1H-benzimidazole (INN: lansoprazole) and 2-{[4-(3-methoxypropoxy)-3-methylpyridin-2-yl]-methylsulfinyl}-1H-benzimidazole (INN: rabeprazole). Further acid-labile proton pump inhibitors, for example substituted phenylmethylsulfinyl-1H-benzimidazoles, cycloheptapyridin-9-ylsulfinyl-1H-benzimidazoles or pyridin-2-ylmethylsulfinylthienoimidazoles, are disclosed in DE-A-35 31 487, EP-A-0 434 999 and EP-A-0 234 485. Examples which may be mentioned are 2-[2-(N-isobutyl-N-methylamino)benzylsulfinyl]benzimidazole (INN: leminoprazole) and 2-(4-methoxy-6,7,8,9-tetrahydro-5H-cyclohepta-[b]pyridin-9-ylsulfinyl)-1H-benzimidazole (INN: nepaprazole).

The acid-labile proton pump inhibitors are chiral compounds. The term acid-labile proton pump inhibitor also includes the pure enantiomers of the acid-labile proton pump inhibitors and their mixtures in any mixing ratio. Pure enantiomers which may be mentioned by way of example are 5-methoxy-2-[(S)-[(4-methoxy-3,5-dimethyl-2-pyridinyl)methyl]sulfinyl]-1H-benzimidazole (INN: esomeprazole).

The acid-labile proton pump inhibitors are present here as such or preferably in the form of their salts with bases. Examples of salts with bases which may be mentioned are sodium, potassium, magnesium and calcium salts. If desired, the salts of the acid-labile proton pump inhibitors with bases can also be present in hydrate form. Particularly preferred acid-labile proton pump inhibitors besides pantoprazole, which may be mentioned are omeprazole magnesium, omeprazole, esomeprazole magnesium and esomeprazole.

In addition to the acid labile proton pump inhibitor (e.g. pantoprazole) the pharmaceutical product may contain one or more additional pharmaceutical acceptable excipients. Examples, which may be mentioned in connection with the invention include complexing agents such as ethylenediamine tetraacetic acid and/or a suitable saft thereof, stabilizers (such as glycine), suitable bases such as sodium carbonate or sodium hydroxide and carriers such as carbohydrates.

The pharmaceutical product according to the invention can be obtained preferably by providing a mixture of the acid labile proton pump inhibitor (e.g. pantoprazol) with a suitable solvent (preferably a solution in an aqueous solvent) and optionally further excipients in the container and subjecting the container comprising the acid labile proton pump inhibitor (e.g. pantoprazole) solution to a freeze drying process. The freeze drying may be carried out by a method known per se. In a preferred embodiment the container is semi-stoppered before applying the freeze-drying process. After finishing the freeze-drying process the container is closed with the stopper preferably under an inert atmosphere (e.g. nitrogen) and reduced pressure and preferably a crimp seal is provided.

According to one embodiment of the invention a pantoprazole solution used in the freeze drying process can be obtained by addition of ethylenediamine tetraacetic acid and/or a suitable salt thereof, and sodium hydroxide and/or sodium carbonate to an aqueous solvent. Suitable salts of ethylenediamine tetraacetic acid which may be mentioned in connection with the invention by way of example are ethylenediamine tetraacetic acid disodium salt, ethylenediamine tetraacetic acid calcium disodium salt ethylenediamine tetraacetic acid trisodium salt and ethylenediamine tetraacetic acid tetrasodium salt. The proportion by weight of ethylenediamine tetraacetic acid and/or a suitable salt thereof, based on the amount of pantoprazole used is from 0.05 to 25 % preferably from 0.25 to 12.5 % or particular preferred from 1 to 5 %. The aqueous solvent preferentially is water for injection. Subsequently pantoprazole is added to the solution and dissolved by stirring. It is preferred to have a solution wherein the proportion of weight (m/m) of pantoprazole is 0.5 to 10 %, particularly preferred 1 to 6 %. In a further preferred embodiment of the invention the pH of the solution used in the freeze drying process is 8 or above 8, particularly preferred the pH is in the range from 10 to 13. Then this solution is filtered for sterilization and charged in vials. The solution is then freeze-dried as described above.

A pantoprazole injection can be produced by dissolving the lyophilized product thus obtained in a suitable solvent for example physiological saline, aqueous solution of 5% glucose, or distilled water for injection. Preferably the pantoprazole injection according to the invention is used in the form of intravenous injection.

The pharmaceutical product according to the invention preferably contains the acid labile proton pump inhibitor (e.g. pantoprazole) in the dose customary for the treatment of the respective disease. The pharmaceutical product according to the invention can be employed for the treatment and prevention of all the diseases, which are regarded as treatable or avoidable by the use of pyridin-2-ylmethylsulfinyl-1H-benzimidazoles. In particular, the pharmaceutical product according to the invention can be employed in the treatment of stomach disorders. Examples which may be mentioned in connection with the invention are the treatment or prophylaxis of benign gastric ulcer, gastroesophageal reflux disease (GERD), GERD associated with a history of erosive esophagitis, pathological hypersecretion associated with Zollinger-Ellison Syndrome, Zollinger-Ellison syndrome, duodenal ulcer, duodenal ulcer associated with Helicobacter pylori, prophylaxis of NSAID-associated gastric or duodenal ulcer in patients with an increased risk of gastroduodenal complication who require continued NSAID treatment or combination therapy with antibiotics in the eradication of Helicobacter pylori. The lyophilized products contained within the pharmaceutical product according to the invention in particular contain between 5 and 150 mg, preferably between 5 and 60 mg, of pantoprazole. Examples, which may be mentioned are lyophilized products or injections which contain 10, 20, 40, 50, 80 or 96 mg of pantoprazole. The administration of the daily dose (e.g. 40 mg of active compound) can be carried out, for example, in the form of an individual dose or by means of a number of doses (e.g. 2 times 20 mg of active compound). The concentration of pantoprazole in the injection may vary depending upon the administration route and generally ranges in a proportion of 0.05-10 mg/ml, preferably 0.1 to 5 mg/ml on a free compound basis. For example for bolus administration 20 to 120 mg of lyophilized product can be reconstituted with 10 ml physiological saline and administered intravenously for example over a period of at least two minutes. In an alternative the contents of two vials (each reconstituted for example with 10 ml of physiological saline) can be combined and further diluted (admixed) with (for example 80 ml) of 5% Dextrose Injection (USP), 0.9% Sodium Chloride Injection (USP) or Lactated Ringer's Injection (USP) (to a total volume of 100 ml). Such solution can be administered intravenously over a period of approximately 15 minutes at a rate of approximately 7 ml/min.

The pharmaceutical product according to the invention may be provided in the form of multiple dose containers, preferably in the form of a single dose container.

The production of the pharmaceutical product according to the invention is described by way of example below. The following examples illustrate the invention in greater detail, without restricting it.

### Examples

### Production of a pharmaceutical product for injection

### Example 1

Under nitrogen atmosphere, 0.276 g Ethylenediamine tetraacetic acid disodium salt and 6.7 g sodium hydroxide (1N aqueous solution) are added to 480 g water for injection of 4°C to 8°C. 12.47 g pantoprazole sodium sesquihydrate is added while stirring to give a clear solution. The weight of the solution is adjusted to 500 g by addition of water for injection. The pH of the solution is 11.76. The solution is filtered through a 0.2 µm membrane filter and filled in glass vials (1.81 g by vial; glass vial of type I glass according to European Pharmacopoeia having a nominal content of 12 ml - Fiolax®). Filled vials are semi-stoppered (type 1 butyl rubber stopper according to European Pharmacopoeia 2002; nominal size 20) and put into a freeze-dryer (e.g. GT4 Edwards/Kniese or GT8 Amsco) for lyophilisation. The vials are cooled to -45°C, then the temperature is raised to -20 to -5°C under vacuum (0.1 to 0.5 mbar) for drying. After finishing main drying the temperature is raised to 30°C, the vacuum is adjusted to 0.01 mbar and drying is continued for an additional 3 hours. The pressure is raised to 500 mbar by flushing with nitrogen and the vials are stoppered and crimped. An off-white lyophilized product is obtained which is easily reconstituted with physiological saline to give a clear solution.

### Example 2

Under nitrogen atmosphere, 12.47 g pantoprazole sodium sesquihydrate is added to 480 g water for injection of 4°C to 8°C while stirring to give a clear solution. The volume of the solution is adjusted to 500 g by addition of water for injection. The pH of the solution is 10.85. The solution is filtered through a 0.2 µm membrane filter and filled in glass vials (1.81 g by vial; glass vial of type I glass according to European Pharmacopoeia having a nominal content of 12 ml - Fiolax®). Filled vials are semi-stoppered (type 1 butyl rubber stopper according to European Pharmacopoeia 2002; nominal size 20) and put into a freeze-dryer (e.g. GT4 Edwards/Kniese or GT8 Amsco) for lyophilisation. The vials are cooled to -45°C, then the temperature is raised to -20 to -5°C under vacuum (0.1 to 0.5 mbar) for drying. After finishing main drying the temperature is raised to 30°C, the vacuum is adjusted to 0.01 mbar and drying is continued for an additional 3 hours. The pressure is raised to 500 mbar by flushing with nitrogen and the vials are stoppered and crimped. An off-white lyophilized product is obtained.

### Example 3

Under nitrogen atmosphere, 2.45 g sodium hydroxide (1N aqueous solution) is added to 480 g water for injection of 4°C to 8°C. 12.47 g pantoprazole sodium sesquihydrate is added while stirring to give a clear solution. The weight of the solution is adjusted to 500 g by addition of water for injection. The pH of the solution is 11.5. The solution is filtered through a 0.2 µm membrane filter and filled in glass vials (1.81 g by vial; glass vial of type I glass according to European Pharmacopoeia having a nominal content of 12 ml - Fiolax®). Filled vials are semi-stoppered (type 1 butyl rubber stopper according to European Pharmacopoeia 2002; nominal size 20) and put into a freeze-dryer (e.g. GT4 Ed-wards/Kniese or GT8 Amsco) for lyophilisation. The vials are cooled to -45°C, then the temperature is raised to -20 to -5°C under vacuum (0.1 to 0.5 mbar) for drying. After finishing main drying the temperature is raised to 30°C, the vacuum is adjusted to 0.01 mbar and drying is continued for an additional 3 hours. The pressure is raised to 500 mbar by flushing with nitrogen and the vials are stoppered and crimped. An off-white lyophilized product is obtained.

### Example 4

Under nitrogen atmosphere, 0.05 g Ethylenediamine tetraacetic acid disodium salt is added to 480 g water for injection of 4°C to 8°C. 12.47 g pantoprazole sodium sesquihydrate is added while stirring to give a clear solution. The weight of the solution is adjusted to 500 g by addition of water for injection. The pH of the solution is 10.2. The solution is filtered through a 0.2 µm membrane filter and filled in glass vials (1.81 g by vial; glass vial of type I glass according to European Pharmacopoeia having a nominal content of 12 ml - Fiolax®). Filled vials are semi-stoppered (type 1. butyl rubber stopper according to European Pharmacopoeia 2002; nominal size 20) and put into a freeze-dryer (e.g. GT4 Edwards/Kniese or GT8 Amsco) for lyophilisation. The vials are cooled to -45°C, then the temperature is raised to -20 to -5°C under vacuum (0.1 to 0.5 mbar) for drying. After finishing main drying the temperature is raised to 30°C, the vacuum is adjusted to 0.01 mbar and drying is continued for an additional 3 hours. The pressure is raised to 500 mbar by flushing with nitrogen and the vials are stoppered and crimped. An off-white lyophilized product is obtained.

### Description of Figures

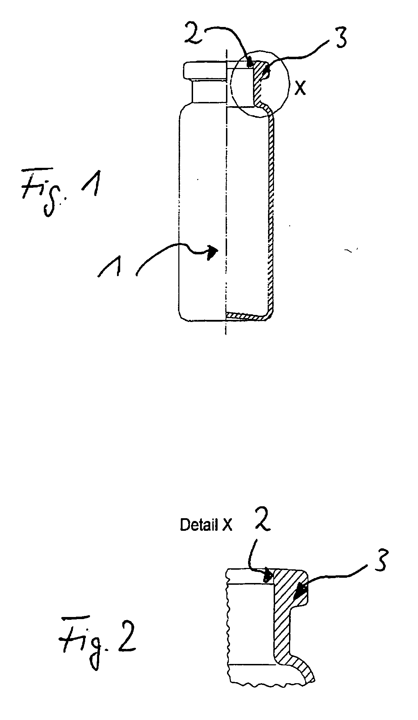

Fig. 1 shows a glass vial (1) with a blow back (2) at the flange (3).
Fig. 2 shows details of the blow back (2) of the vial (1)

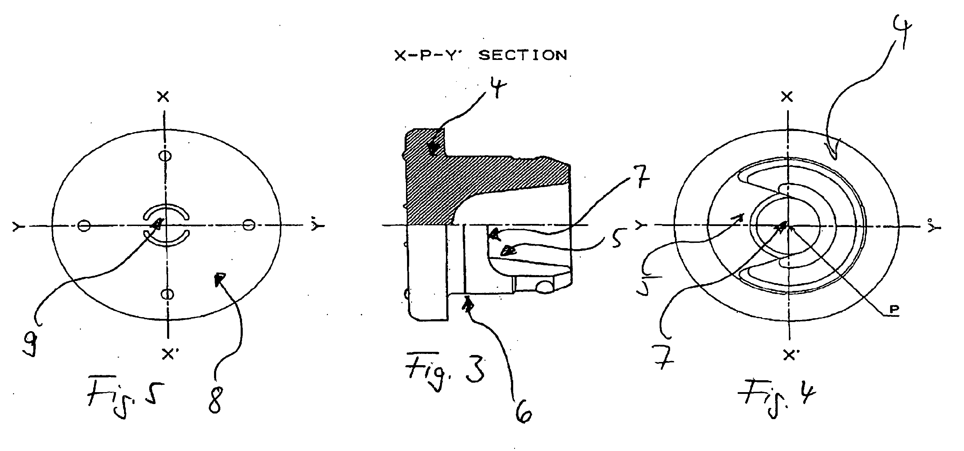

Fig. 3 shows details of a rubber stopper (4). The stopper is provided with a slit (5). The areas of the plug part starting with the line indicated by (6) downwards and inside the plug part (7) are laminated with a fluoro-polmer.
Fig. 4 shows a downside view of the rubber stopper (4).
Fig. 5 shows the topside view of the rubber stopper (5) with the top surface of the flange part (8), having a mark (9) for the injection site.

## Claims

1. Pharmaceutical product for injection comprising a container including a closure suitable for preparations for injection, the container containing a compound selected from the group of 5-difluoromethoxy-2-[(3,4-dimethoxy-2-pyridinyl)methylsulfinyl]-1H-benzimidazole (pantoprazole), a salt thereof, a solvate of pantoprazole and a salt thereof, wherein the container and closure are made of material which essentially does not release zinc ions, whereby the closure is a rubber stopper of type 1 according to European Pharmacopoeia 2002, wherein the amount of extractable zinc from the closure is 0 ppm (i.e. not detectable), when determined according to European Pharmacopeia 2002..

2. Pharmaceutical product according to claim 1, wherein pantoprazole is pantoprazole sodium sesquihydrate.

3. Product according to any of claims 1 or 2, wherein the closure is a freeze-drying closure.

4. Product according to claim 1, wherein the closure is a buty rubber stopper of type 1 according to European Pharmacopeia 2002, which is partially fluoro-polymer laminated.

5. Product according to claim 1, comprising a clear glass vial fitted with a rubber stopper of type 1 according to European Pharmacopoeia 2002 and a crimp seal.

6. Product according to claim 5, wherein the glass vial comprises a blow back inside the flange, and the closure is a butyl rubber stopper of type 1 according to European Pharmacopoeia 2002 having 0 ppm of extractable zinc, which stopper is partially fluoro-polymer laminated.

7. Product according to claim 6, wherein the fluoro-polymer lamination extends from the area of the stopper surface following the area of the stopper which is contacting the blow back inside the flange of the vial downwards and covers those parts of the stopper which extend inside the vial.

8. Product according to claim 1, having reduced pressure inside the container.

9. Product according to claim 8, wherein the reduced pressure is 800 mbar or below, 600 mbar or below or 500 mbar or below.

10. Product according to any of claims 1 to 9, having a volume of 20 ml or less.

11. Product according to any of claims 1 to 10, additionally containing one or more suitable excipients.

12. Product according to claim 11, wherein the excipients are selected from the group of complexing agents, stabilizers, suitable bases, carriers and mixtures thereof.

13. Product according to claim 12, comprising ethylendiamine tetraacetic acid and/or a suitable salt thereof and sodium hydroxide.

14. Process for manufacturing a pharmaceutical product for injection according to claim 1 comprising the steps of (a) providing a mixture of a compound selected from the group of 5-difluoro-methoxy-2-[(3,4-dimethoxy-2-pyridinyl)methylsulfinyl]-1H-benzimidazole (pantoprazole), a salt thereof, a solvate of pantoprazole and a salt thereof, with a solvent and optionally further excipients in the container (b) subjecting the container comprising the above mixture to freeze drying and (c) closing the container with the closure.

15. Process according to claim 14, wherein step (c) is affected under reduced pressure.

## Patentansprüche

1. Pharmazeutisches Produkt für die Injektion, umfassend einen Behälter einschließlich eines Verschlusses, der für Präparate für die Injektion geeignet ist, wobei der Behälter eine Verbindung enthält, ausgewählt aus der Gruppe von 5-Difluormethoxy-2-[(3,4-dimethoxy-2-pyridinyl)methylsulfinyl]-1H-benzimidazol (Pantoprazol), einem Salz davon, einem Solvat von Pantoprazol und einem Salz davon, wobei der Behälter und der Verschluss aus einem Material hergestellt sind, das im Wesentlichen keine ZinkIonen freisetzt, wobei der Verschluss ein Gummistopfen vom Typ 1 gemäß dem Europäischen Arzneibuch von 2002 ist, wobei die Menge an aus dem Verschluss extrahierbarem Zink bei Bestimmung gemäß dem Europäischen Arzneibuch von 2002 0 ppm (d. h. nicht nachweisbar) beträgt.

2. Pharmazeutisches Produkt gemäß Anspruch 1, wobei Pantoprazol Pantoprazol-Natriumsesquihydrat ist.

3. Produkt gemäß einem der Ansprüche 1 oder 2, wobei der Verschluss ein Gefriertrocknungsverschluss ist.

4. Produkt gemäß Anspruch 1, wobei der Verschluss ein Butylgummistopfen vom Typ 1 gemäß dem Europäischen Arzneibuch von 2002 ist, der teilweise Fluorpolymer-laminiert ist.

5. Produkt gemäß Anspruch 1, umfassend ein klares Glasfläschchen, das mit einem Gummistopfen vom Typ 1 gemäß dem Europäischen Arzneibuch von 2002 und einem Bördelverschluss versehen ist.

6. Produkt gemäß Anspruch 5, wobei das Glasfläschchen ein Blowback innerhalb des Flanschs aufweist und der Verschluss ein Butylgummistopfen vom Typ 1 gemäß dem Europäischen Arzneibuch von 2002 mit 0 ppm extrahierbarem Zink ist, welcher Stopfen teilweise Fluorpolymer-laminiert ist.

7. Produkt gemäß Anspruch 6, wobei sich die Fluorpolymer-Lamination von dem Bereich der Stopfenoberfläche, die dem Bereich des Stopfens folgt, der das Blowback innerhalb des Flanschs des Fläschchens kontaktiert, abwärts erstreckt und die Teile des Stopfens bedeckt, die sich ins Innere des Fläschchens erstrecken.

8. Produkt gemäß Anspruch 1 mit verringertem Druck innerhalb des Behälters.

9. Produkt gemäß Anspruch 8, wobei der verringerte Druck 800 mbar oder darunter, 600 mbar oder darunter oder 500 mbar oder darunter beträgt.

10. Produkt gemäß einem der Ansprüche 1 bis 9, das ein Volumen von 20 ml oder weniger aufweist.

11. Produkt gemäß einem der Ansprüche 1 bis 10, das zusätzlich einen oder mehrere geeignete Hilfsstoffe enthält.

12. Produkt gemäß Anspruch 11, wobei die Hilfsstoffe ausgewählt sind aus der Gruppe von Komplexierungsmitteln, Stabilisatoren, geeigneten Basen, Trägern und Gemischen davon.

13. Produkt gemäß Anspruch 12, umfassend Ethylendiamintetraessigsäure und/oder ein geeignetes Salz davon und Natriumhydroxid.

14. Verfahren zum Herstellen eines pharmazeutischen Produkts für die Injektion gemäß Anspruch 1, umfassend die Schritte (a) Versehen eines Gemischs einer Verbindung, ausgewählt aus der Gruppe von 5-Difluormethoxy-2-[(3,4-dimethoxy-2-pyridinyl)methylsulfinyl]-1H-benzimidazol (Pantoprazol), einem Salz davon, einem Solvat von Pantoprazol und einem Salz davon, mit einem Lösungsmittel und gegebenenfalls weiteren Hilfsstoffen in dem Behälter, (b) Unterwerfen des Behälters, der das vorstehend genannte Gemisch umfasst, einer Gefriertrocknung, und (c) Verschließen des Behälters mit dem Verschluss.

15. Verfahren gemäß Anspruch 14, wobei Schritt (c) unter verringertem Druck durchgeführt wird.

## Revendications

1. Produit pharmaceutique pour injection comprenant un récipient comprenant une fermeture adaptée pour des préparations pour injection, le récipient contenant un composé choisi dans le groupe du 5-difluorométhoxy-2-[(3,4-diméthoxy-2-pyridinyl)méthylsulfinyl]-1H-benzimidazole (pantoprazole), un sel de celui-ci, un solvate de pantoprazole et un sel de celui-ci, dans lequel le récipient et la fermeture sont constitués d'un matériau qui ne libère pratiquement pas d'ions de zinc, la fermeture étant un bouchon en caoutchouc de type 1 selon la Pharmacopée européenne 2002, dans lequel la quantité de zinc extractible de la fermeture est de 0 ppm (c'est-à-dire non détectable), lorsqu'elle est déterminée selon la Pharmacopée européenne 2002.

2. Produit pharmaceutique selon la revendication 1, dans lequel le pantoprazole est du pantoprazole de sodium sesquihydraté.

3. Produit selon l'une quelconque des revendications 1 ou 2, dans lequel la fermeture est une fermeture de lyophilisation.

4. Produit selon la revendication 1, dans lequel la fermeture est un bouchon en caoutchouc butyle de type 1 selon la Pharmacopée européenne 2002, qui est partiellement laminé avec du fluoropolymère.

5. Produit selon la revendication 1, comprenant un flacon de verre équipé d'un bouchon de caoutchouc de type 1 selon la Pharmacopée européenne 2002 et d'un joint serti.

6. Produit selon la revendication 5, dans lequel le flacon en verre comprend un refoulement à l'intérieur de la bride, et la fermeture est un bouchon en caoutchouc butyle de type 1 selon la Pharmacopée européenne 2002 ayant 0 ppm de zinc extractible, ledit bouchon étant partiellement laminé avec du fluoropolymère.

7. Produit selon la revendication 6, dans lequel le laminage de fluoropolymère s'étend de la zone de la surface du bouchon suivant la zone du bouchon qui est en contact avec le refoulement à l'intérieur de la bride du flacon vers le bas et couvre les parties du bouchon qui s'étendent à l'intérieur du flacon.

8. Produit selon la revendication 1, ayant une pression réduite à l'intérieur du récipient.

9. Produit selon la revendication 8, dans lequel la pression réduite est de 800 mbar ou moins, 600 mbar ou moins ou 500 mbar ou moins.

10. Produit selon l'une quelconque des revendications 1 à 9, ayant un volume de 20 ml ou moins.

11. Produit selon l'une quelconque des revendications 1 à 10, contenant en outre un ou plusieurs excipients adaptés.

12. Produit selon la revendication 11, dans lequel les excipients sont choisis dans le groupe d'agents complexants, stabilisants, bases adaptées, véhicules et mélanges de ceux-ci.

13. Produit selon la revendication 12, comprenant de l'acide éthylènediaminetétraacétique et/ou un sel adapté de celui-ci et de l'hydroxyde de sodium.

14. Procédé pour fabriquer un produit pharmaceutique pour injection selon la revendication 1 comprenant les étapes de (a) fourniture d'un mélange d'un composé choisi dans le groupe du 5-difluoro-méthoxy-2-[(3,4-diméthoxy-2-pyridinyl)méthylsulfinyl]-1H-benzimidazole (pantoprazole), un sel de celui-ci, un solvate de pantoprazole et un sel de celui-ci, avec un solvant et facultativement en outre des excipients dans le récipient, (b) soumission du récipient comprenant le mélange ci-dessus à une lyophilisation et (c) fermeture du récipient avec la fermeture.

15. Procédé selon la revendication 14, dans lequel l'étape (c) est effectuée sous pression réduite.
